(19) Europäisches Patentamt
European Patent Office
Office européen des brevets

(11) **EP 2 660 573 A2**

(12) **EUROPEAN PATENT APPLICATION**

(43) Date of publication:
**06.11.2013 Bulletin 2013/45**

(51) Int Cl.:
**G01J 3/28** (2006.01)      **G01J 3/457** (2006.01)
**G06F 19/00** (2011.01)

(21) Application number: **13002381.5**

(22) Date of filing: **03.05.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **04.05.2012  US 201213464278**

(71) Applicant: **Morpho Detection, Inc.**
**Newark, CA 94560 (US)**

(72) Inventors:
• **Vignesh, Thirukazhukundram Subrahmaniam**
**560037 Bangalore (IN)**

• **Sutherland, William Scott**
**Spring, TX 77379 (US)**
• **Lee, Young Kyo**
**San Diego, CA 92104 (US)**
• **Dasaratha, Sridhar Venkataraman**
**560066 Bangalore (IN)**

(74) Representative: **Müller-Boré & Partner**
**Patentanwälte**
**Grafinger Straße 2**
**81671 München (DE)**

(54) **Systems and methods for identifying a plurality of compounds in a mixture**

(57)      A spectrometer for identifying a mixture is provided. The spectrometer includes a detector configured to generate a signal based on an interaction of light with a sample of the mixture, and a memory device having a library and a correlation matrix stored therein, wherein the library includes a plurality of spectra, each spectrum associated with a respective compound, and wherein the correlation matrix includes a correlation between each possible pair of spectra in the library. The spectrometer further includes a processor coupled to the memory device and configured to determine a spectrum of the mixture based on the signal generated by the detector, calculate a correlation vector that includes a correlation between the mixture spectrum and each spectrum in the library, and identify the mixture based on the correlation matrix and the correlation vector.

FIG. 1

**Description**

BACKGROUND OF THE INVENTION

The embodiments described herein relate generally to spectroscopy systems and, more particularly, to identifying a plurality of compounds in a mixture.

Rapid identification of unknown materials has emerged as an important problem in a variety of situations such as quality control, failure analysis, clinical assays, and material analysis involving hazardous materials. For example, the quality of a product, such as a drug, is dependent on the purity of the raw materials used, and any contamination within the raw materials may be detrimental to the quality and/or efficacy of the product. As such, identifying the contaminants is important in such situations. Moreover, analytical techniques may also be applied to detect a chemical change in the structure of a material that may lead to failure of critical parts or components in, for example, gas turbine engines. Another application involves identification of unknown materials that are potentially hazardous in nature.

Analytical techniques using spectroscopy have been used in such situations. At least some known spectrometry instruments include a search engine that returns a list of chemicals or compounds of a sample and, for example, a Euclidean distance, correlation, and the like. For example, at least some known spectrometers identify compounds of a mixture by comparing a spectrum of the mixture to a plurality of spectra that are each associated with a different compound. Moreover, at least some known spectrometers use linear models, mathematical analyses such as an augmented least squares analysis, and/or a state matrix to identify compounds of a mixture. In addition, at least some known spectrometers use scaling factors and threshold values to facilitate identifying compounds of a mixture.

However, at least some known spectroscopy methods analyze samples using algorithms that may be relatively computationally intensive. In general, the more accurate the identification algorithm, the more computational resources and/or time the algorithm may require to identify the material. Accordingly, due to computational and/or time constraints, at least some known spectrometers employ less accurate algorithms to reduce the processing power and/or time required to analyze a sample.

BRIEF SUMMARY OF THE INVENTION

In one aspect, a spectrometer for identifying a mixture is provided. The spectrometer includes a detector configured to generate a signal based on an interaction of light with a sample of the mixture, and a memory device having a library and a correlation matrix stored therein, wherein the library includes a plurality of spectra, each spectrum associated with a respective compound, and wherein the correlation matrix includes a correlation between each possible pair of spectra in the library. The spectrometer further includes a processor coupled to the memory device and configured to determine a spectrum of the mixture based on the signal generated by the detector, calculate a correlation vector that includes a correlation between the mixture spectrum and each spectrum in the library, and identify the mixture based on the correlation matrix and the correlation vector.

In another aspect, a processing device is provided. The processing device is configured to acquire a spectrum of a mixture, calculate a correlation vector that includes a correlation between the mixture spectrum and each of a plurality of spectra stored in a library, and identify the mixture based on the correlation vector and a correlation matrix that includes a correlation between each possible pair of spectra in the library.

In yet another aspect, a method for identifying a mixture is provided. The method includes acquiring, using a spectrometer, a spectrum of the mixture, calculating, using a processing device, a correlation vector that includes a correlation between the mixture spectrum and each of a plurality of spectra stored in a library, each library spectrum associated with a respective compound, and identifying, using the processing device, the mixture based on the correlation vector and a correlation matrix that includes a correlation between each possible pair of spectra in the library.

BRIEF DESCRIPTION OF THE DRAWINGS

Fig. 1 is a schematic diagram of an exemplary spectrometer.

Fig. 2 is a schematic block diagram of an exemplary optical architecture that may be used with the spectrometer shown in Fig. 1.

Fig. 3 is a schematic block diagram of an exemplary electrical architecture that may be used with the spectrometer shown in Fig. 1.

Fig. 4 is a flowchart of an exemplary method for identifying a plurality of compounds in a mixture using a subtraction algorithm.

Fig. 5 is a schematic diagram illustrating operation of the subtraction algorithm shown in Fig. 4.

Fig. 6 is a flowchart of an exemplary method for identifying an unknown mixture using a mean squared error algorithm.

Fig. 7 is a flowchart of a method for calculating the mean squared error of each fit for a plurality of multi-compound models.

Fig. 8 is a flowchart of an exemplary method for calculating the mean squared error of each fit utilizing a correlation matrix.

DETAILED DESCRIPTION OF THE INVENTION

The systems and methods described herein enable identification of a mixture using a correlation matrix. By utilizing a correlation matrix, the number of calculations required by a mixture identification algorithm may be significantly reduced, enabling identification of mixtures in less time and with fewer computational resources. That is, floating point and/or intermediary computations required by at least some known mixture identification algorithms can be eliminated by using the correlation matrix. Accordingly, the embodiments described herein provide relatively efficient and fast analysis of mixtures.

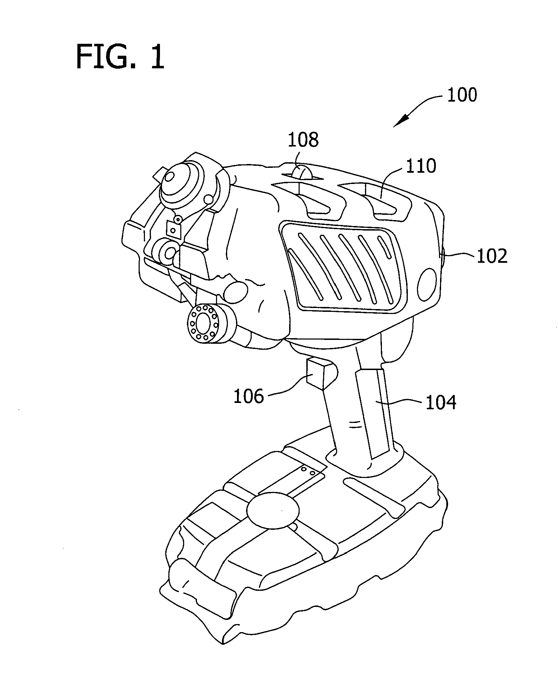

Fig. 1 is a schematic diagram of an exemplary portable, handheld spectrometer 100 for use in analyzing a mixture to determine one or more possible compounds in the mixture. Although Fig. 1 describes a portable spectrometer, it should be understood that the systems and methods described herein are not limited to use on portable or handheld spectrometers or devices. Rather, the methods described herein may be practiced using stationary devices or using portable devices that are not handheld. Spectrometer 100 may be used to analyze and identify a wide variety of materials, including, but not limited to, narcotics, explosives, poisons, toxic chemicals, and/or hazardous materials. For example, spectrometer 100 may be utilized by first responders at an accident and/or incident site to identify unknown materials. Spectrometer 100 may also be used in security environments such as airports, prisons, or border crossings to identify unknown materials.

In the exemplary embodiment, spectrometer 100 includes a main body 102 and a handle 104 that is coupled to the main body 102. Handle 104 includes an input device 106 that initiates operation of spectrometer 100 as described in greater detail below. In the exemplary embodiment, input device 106 is a trigger. However, input device 106 may be any suitable means for receiving a user input such as, but not limited to, a sliding switch, a toggle switch, or a button. Moreover, in the exemplary embodiment, main body 102 includes one or more user control devices 108 such as, but not limited to, a joystick. Main body 102 also includes a display device 110 that displays, for example, a spectrum acquired from the mixture and/or a list that includes the plurality of possible compounds within the mixture.

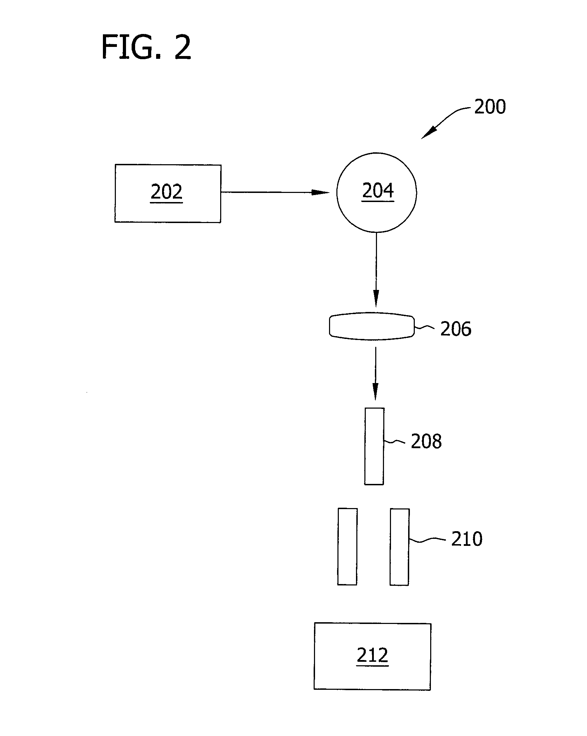

Fig. 2 is a schematic block diagram of an exemplary optical architecture 200 of spectrometer 100 (shown in Fig. 1). In the exemplary embodiment, optical architecture 200 is positioned within main body 102 (shown in Fig. 1). Moreover, in the exemplary embodiment, optical architecture 200 includes an optical source 202, such as a laser that emits a monochromatic light beam in a visible light range, a near infrared light range, an infrared light range, a fluorescent light range, and/or an ultraviolet light range. Specifically, optical source 202 directs incident photons at a sample 204 of the mixture to be identified. In the exemplary embodiment, sample 204 emits Raman scattered light in response to the photons at an angle with respect to a path of the incident photons. The scattered light is collected using a lens 206, which is positioned to adjust a focal spot and to enhance a signal strength of the scattered light. Lens 206 is coupled to a Fiber Bragg grating (FBG) 208 via an optical fiber (not shown) to facilitate channeling the scattered light to FBG 208. In some embodiments, FBG 208 has a fixed transmission wavelength that is based on a pitch of FBG 208. In the exemplary embodiment, the scattered light is channeled through a tunable Fabry-Perot cavity 210 towards a sample detector 212. Optical architecture 200 may be calibrated using, for example, an argon lamp.

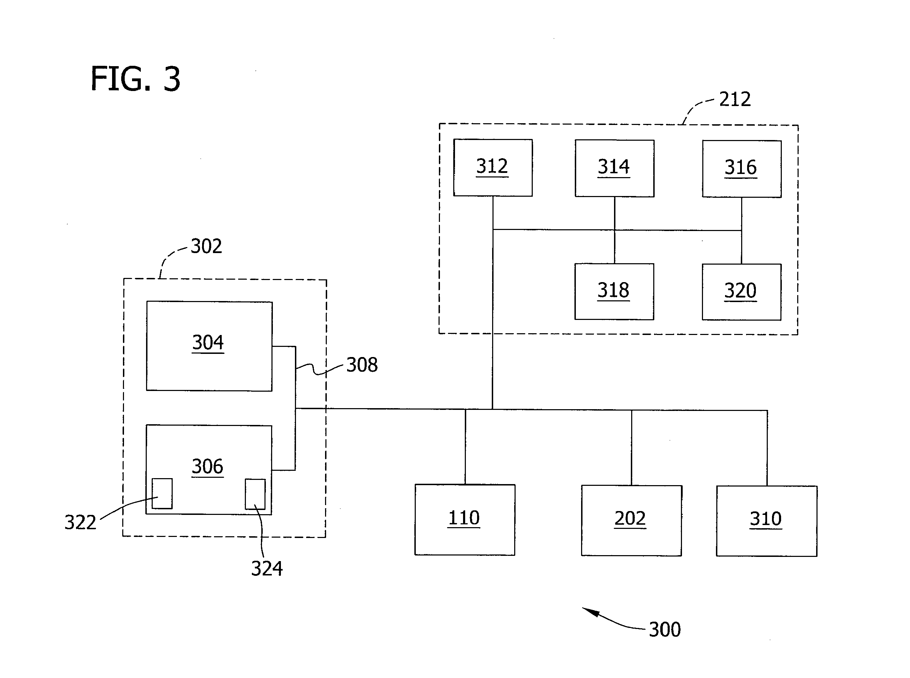

Fig. 3 is a schematic block diagram of an exemplary electrical architecture 300 of spectrometer 100 (shown in Fig. 1). In the exemplary embodiment, spectrometer 100 includes a controller 302 that includes a processor 304 and a memory 306 that is coupled to processor 304 via an address/data bus 308. Alternative embodiments of controller 302 may include more than one processor 304, memory modules 306, and/or different types of memory modules 306. For example, memory 306 may be implemented as, for example, semiconductor memories, magnetically readable memories, optically readable memories, or some combination thereof. In some embodiments, controller 302 is coupled to a network (not shown) via a network interface 310.

Moreover, in the exemplary embodiment, electrical architecture 300 includes optical source 202 and sample detector 212. Sample detector 212 includes an avalanche photodiode (APD) 312, a discriminator 314, a digitizer 316, and one or more amplifiers, such as a preamplifier 318 and a high-gain amplifier 320. Raman scattered light emitted by sample 204 (shown in Fig. 2) is incident upon APD 312. In response to the Raman scattered light, APD 312 outputs a current pulse to preamplifier 318, which shapes the pulse to create a Nuclear Instrumentation Methods (NIM) standard current pulse. Amplifier 320 receives the NIM pulse, and converts the NIM pulse into a voltage signal.

Discriminator 314 receives the amplified voltage signal from amplifier 320, and isolates single photon signals that correspond to voltage pulses within a specified range. Discriminator 314 outputs an analog signal based on the isolated single photon signals. Digitizer 316 converts the analog signal into a digital signal. Processor 304 determines a spectrum for sample 204 based on the digital signal. In some embodiments, processor 304 causes display device 110 to display the spectrum to a user. The spectrum may also be stored in memory 306 for retrieval by processor 304.

Before implementing algorithms (such as those described in detail below) to identify spectrum, and accordingly sample 204, the spectrum may be corrected and/or pre-processed to remove extraneous signals and/or artifacts in the spectrum. Such signals and/or artifacts may be present due to various instrumental effects, such as, but not limited to, the transmission of optical elements, the variability of detector response, and/or other effects. For example, in Raman spectroscopy, fluorescence and baseline artifacts may be present in the initial spectrum. The spectrum may be pre-processed using, for example, a Savitzky-Golay filter.

In the exemplary embodiment, memory 306 includes a library 322 that stores a plurality of spectra, such as Raman spectra, of a plurality of compounds. Library 322 may be a complete collection of spectra, or only a subset of a larger collection of spectra. Spectra in library may also be preprocessed to remove extraneous signals and/or artifacts. Compounds may be liquid, gas, powder, and/or solid compounds. A correlation matrix 324 is calculated from the spectra in library 322, and stored in memory 306. Correlation matrix 324 is utilized in algorithms for identifying sample 204, as described in detail below.

One or more of the steps of the algorithms described herein may be performed using a processing device, such as processor 304. In some embodiments, one or more of the steps of the algorithms described herein are performed by a remote processing device not located within spectrometer 100 (shown in Fig. 1). For example, spectrometer 100 may transmit a spectrum to a remote computing device, and a processing device onboard the remote computing device may identify compounds in the spectrum using the algorithms described herein.

Mixture Identification using a Subtraction Algorithm

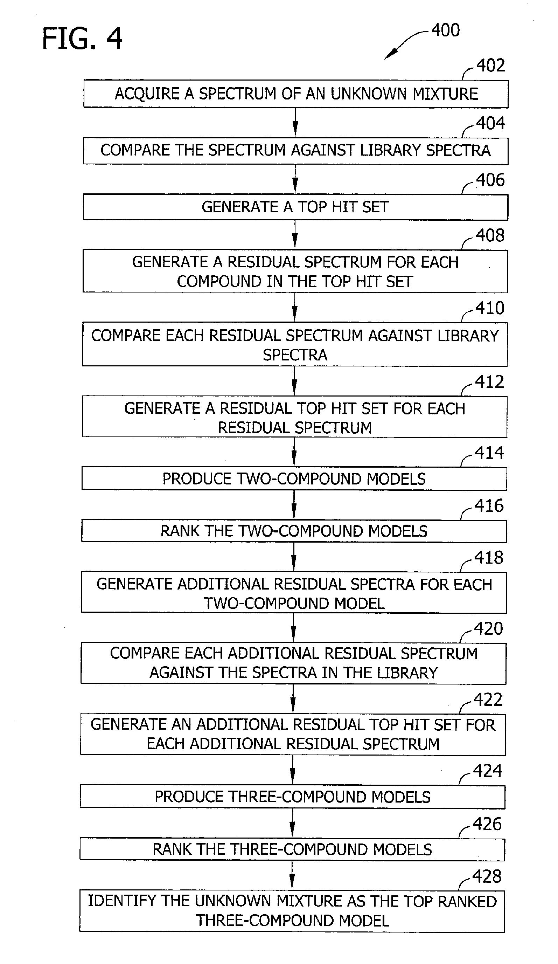

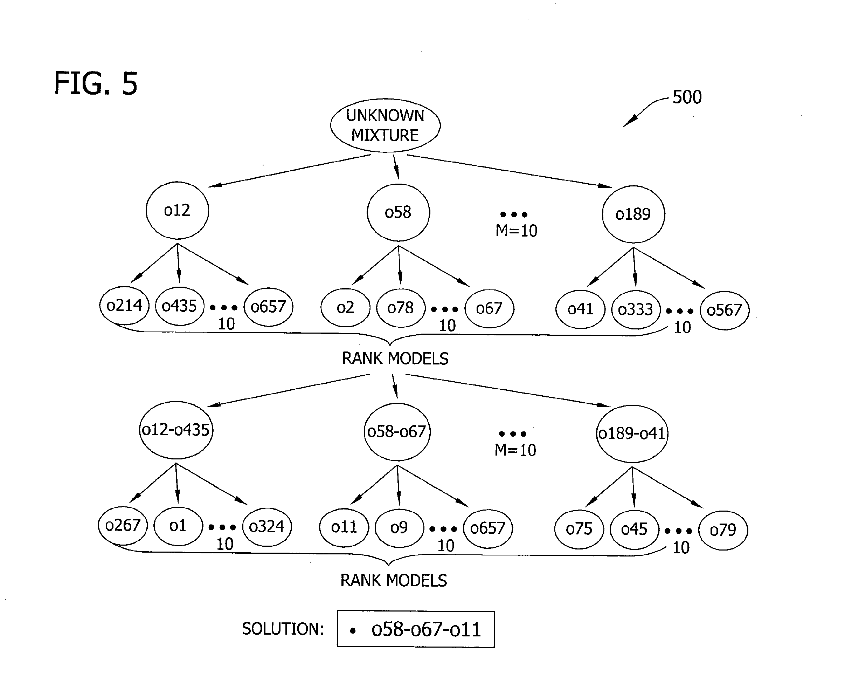

Fig. 4 is a flowchart of an exemplary method 400 for identifying one or more compounds in a mixture, such as sample 204 (shown in Fig. 2), using a three-pass subtraction algorithm. The mixture may include and/or may be identified as a plurality of compounds, or only one compound (i.e., a pure substance). Fig. 5 is a schematic diagram 500 illustrating the operation of the subtraction algorithm. In the exemplary embodiment, the subtraction algorithm performs three passes, identifying a plurality of three-compound models for sample 204, as described in detail below. Alternatively, any suitable number of passes may be performed by the subtraction algorithm. For example, to identify two-compound models, only two passes are performed by the subtraction algorithm. Unless otherwise noted, in the exemplary embodiment, processor 304 (shown in Fig. 3) performs the steps of method 400.

In the exemplary embodiment, spectrometer 100 (shown in Fig. 1) acquires 402 a spectrum, such as a Raman spectrum, of the unknown mixture. For a first pass of the subtraction algorithm, the spectrum is compared 404 against the spectra of compounds in library 322 (shown in Fig. 3) A top hit set, t, that includes the list of compounds in library 322 that have the highest correlation with the spectrum is generated 406. In the exemplary embodiment, top hit set t includes the ten most closely correlated compounds in library 322. Alternatively, top hit set t may include any number of compounds that enables spectrometer 100 to function as described herein. For example, top hit set t may include a specific number of compounds or all compounds having a mean absolute error below a threshold value. In diagram 500, three of the ten compounds in top hit set t are shown (i.e., o12, o58, and o189).

For each compound in top hit set t, a residual spectrum is generated 408 by subtracting the spectrum of the compound from the acquired spectrum. In the second pass of the algorithm, each residual spectrum is then compared 410 against the spectra in library 322 to generate 412 a residual top hit set t' for each residual spectrum. For example, in diagram 500, the residual top hit set t' for the residual spectrum obtained by subtracting the spectrum of o12 from the acquired mixture spectrum includes o214, o435, and o657.

After the second pass, a plurality of two-compound models (e.g., o12-o214, o12-o435, ... o58-067, ... o189-o567) are produced 414 from combinations of the compounds in top hit set t and residual top hit set t'. These two-compound models are ranked 416 according to predetermined criteria. In the exemplary embodiment, the two-compound models are ranked by their respective mean absolute error. Alternatively, the models may be ranked using any suitable measure. At this point, a two-pass subtraction algorithm is complete, and the two-compound model at the top of the rankings is the most likely two-compound combination in the mixture.

For the three-pass subtraction algorithm, the top ranked two-compound models are used to generate 418 additional residual spectra by subtracting the spectrum of each two-compound model from the original mixture spectrum. For example, in diagram 500, the spectrum of the two-compound model of o12 and o435 is subtracted from the acquired mixture spectrum to generate one additional residual spectrum, the spectrum of the two-compound model of o58 and o67 is subtracted from the original mixture spectrum to generate another additional residual spectrum, and the spectrum of the two-compound model of o189 and o41 is subtracted from the original mixture spectrum to generate another additional residual spectrum.

Similar to the second pass, each additional residual spectrum is compared 420 against the spectra in library 322 to generate 422 an additional residual top hit set t" for each additional residual spectrum. For example, in diagram 500, the additional residual top hit set t" for the residual spectrum obtained by subtracting the spectrum of the two-compound model including o12 and o435 from the acquired mixture spectrum includes o267, o1, and o324.

After the third pass, a plurality of three-compound models (e.g., o12-o435-0267) are produced 424 from the two-compound models from the second pass, and the additional residual top hit set t" for each additional residual spectrum. These three-compound models are ranked 426 according to predetermined criteria, and the three-compound model at the top of the rankings is the most likely three-compound combination in the mixture. For example, in diagram 500, the most likely three-compound combination in the mixture is determined to be o58, 067, and o11. Accordingly, the mixture is identified 428 as the top ranked three-compound combination. In the exemplary embodiment, method 400 is a three-pass subtraction method. Alternatively, method may include additional passes or fewer passes (i.e., k passes to identify the mixture as a k-compound mixture).

Notably, comparing 404 the spectrum against the spectra in library 322, comparing 410 each residual spectrum against the spectra in library 322, and comparing 420 each additional residual spectrum against spectra in library 322 may involve a relatively high number of correlation computations. For example, to generate 412 a residual top hits set t' for ten residual spectra by comparing 410 each residual spectrum against a library with spectra for 1000 compounds would require 10,000 correlation computations. However, in the exemplary embodiment, and as described in detail below, correlation matrix 324 (shown in Fig. 3) is utilized to simplify correlation computations, significantly reducing the time and/or processing power needed to implement the subtraction algorithm.

In the exemplary embodiment, suppose library 322 includes N compounds, each having a vector $X_i$ that contains that particular compound's spectral intensity (i.e., its spectrum). Further, for computational ease, assume that each library vector $X_i$ is normalized to unit energy. Further, let y be the normalized vector of the spectral intensity of the unidentified mixture (i.e., the mixture in sample 204 (shown in Fig. 2)). In the exemplary embodiment, the spectrum of each compound in library 322 is normalized to unit energy in a pre-processing step. Alternatively, each library spectrum may be normalized during processing based on a standard deviation of each library spectrum. The correlation operator between two vectors can be expressed using Equation 1:

$$< x, y >= \frac{\sum_i x_i y_i}{\sqrt{\sum_i x_i^2 \sum_i y_i^2}} \qquad (1)$$

When x and y are normalized, $\sum_i x_i^2 = 1$ and $\sum_i y_i^2 = 1$, , and Equation 1 becomes:

$$< x, y >= \sum_i x_i y_i \qquad (2)$$

In the exemplary embodiment, correlation matrix 324 is an NxN correlation matrix R that contains all of the computed correlations between the spectra of any two compounds in library 322. For example, for a library containing four compounds:

$$R = \begin{bmatrix} 1 & <X_1,X_2> & <X_1,X_3> & <X_1,X_4> \\ <X_2,X_1> & 1 & <X_2,X_3> & <X_2,X_4> \\ <X_3,X_1> & <X_3,X_2> & 1 & <X_3,X_4> \\ <X_4,X_1> & <X_4,X_2> & <X_4,X_3> & 1 \end{bmatrix} \qquad (3)$$

Accordingly, R is a symmetric matrix with entries along the diagonal equal to one, and each entry in R is given by Equation 4:

$$R_{ij} = <X_i, X_j> \qquad (4)$$

Notably, the entries in the correlation matrix R can be computed before any mixture spectra are acquired, and the correlation matrix R is the same, regardless of the mixture analyzed. Accordingly, in the exemplary embodiment, when spectrometer 100 (shown in Fig. 1) acquires 402 a spectrum of an unknown mixture, the correlation matrix R may already be computed and stored in memory 306. Alternatively, correlation matrix R may be computed at any time that enables spectrometer 100 to function as described herein, including on the fly during execution of the algorithms described herein. Further, correlation matrix R may be stored in memory 306 and/or stored in a memory device remote from spectrometer 100. Further, in some embodiments, correlation matrix R itself may not be stored, but may be calculated from other stored values, such as, but not limited to, a transformed correlation matrix, a covariance matrix, standard deviation of each spectrum in library 322, and/or an inverse of correlation matrix R. To update correlation matrix R when new spectra are added to library 322, the correlation matrix R may be recomputed on-line (i.e., by processor 304) or recomputed off-line (i.e., by an external processing device) and then loaded onto spectrometer 100. Further, in some embodiments, matrices other than correlation matrix R be utilized. For example, a matrix containing weighted correlations between library spectra or a matrix containing the covariance between library spectra may be utilized.

Let r denote an Nx1 dimensional correlation vector containing the correlations between the spectrum y of the unidentified mixture and each of the N library spectra. That is:

$$r = \begin{bmatrix} <y, X_1> \\ <y, X_2> \\ \vdots \\ <y, X_N> \end{bmatrix} \qquad (5)$$

In the exemplary embodiment, the correlation vector r is calculated during the first pass of the subtraction algorithm, when the spectrum of the unknown mixture is compared against the spectra of all of the compounds in library 322.

By computing the correlation matrix R initially, the number of calculations needed to perform the subtraction algorithm is significantly reduced. For example, as part of the third pass of the subtraction algorithm, processing device 304 compares 420 an additional residual spectrum AddRsid against every compound in library 322 by computing the correlation between the additional residual spectrum AddRsid and every spectra in library 322. If the additional residual spectrum AddRsid is generated 418 using a two-compound model including compound A and compound B (determined from the first and second pass of the subtraction algorithm), AddRsid can be expressed as:

$$AddRsid = y - \alpha_A X_A - \alpha_B X_B \qquad (6)$$

where $\alpha_A$ and $\alpha_B$ are regression coefficients.

The regression coefficients can be calculated using:

$$\begin{bmatrix} \sigma_A \\ \sigma_B \end{bmatrix} = inv\left( \begin{bmatrix} 1 & R_{AB} \\ R_{AB} & 1 \end{bmatrix} \right) \times \begin{bmatrix} r_{Ay} \\ r_{By} \end{bmatrix} \qquad (7)$$

where $R_{AB}$ is the correlation between library spectra corresponding to substances A and B, $r_{Ay}$ is the correlation between the unknown spectrum and the library spectrum corresponding to substance A, $r_{By}$ is the correlation between the unknown spectrum and the library spectrum corresponding to substance B, and inv( ) is the inverse of a matrix which may be calculated using Gaussian Elimination. $R_{AB}$ may either be read from a stored instance of correlation matrix R or computed on the fly as the algorithm is performed.

Because the correlation operator of Equation 1 is linear, the correlation between AddRsid and every compound in the library can be expressed in terms of entries in the correlation matrix R and the correlation vector r of the unidentified mixture by mathematical manipulation. Specifically:

$$< AddRsid, X_i > = < y - \alpha_A X_A - \alpha_B X_B, X_i > \qquad (8)$$

$$< AddRsid, X_i > = < y, X_i > - \alpha_A < X_A, X_i > - \alpha_B < X_B, X_i >$$

$$(9)$$

$$< AddRsid, X_i > = r_i - \alpha_A R_{Ai} - \alpha_B R_{Bi} \qquad (10)$$

Accordingly, the correlation between the additional residual spectrum AddRsid and the spectrum of any compound in library 322 can be calculated using the previously calculated correlations in correlation matrix R and the correlation vector r that is calculated during the first pass of the subtraction algorithm. Further, as the correlation matrix R is symmetric (i.e., $<X_i,X_j> = <X_j,X_i>$), memory 306 may include only one of the upper and lower half of the correlation matrix R. This significantly reduces the number of calculations required to perform the subtraction algorithm.

While Equation 9 applies to the third pass of the subtraction algorithm, similar equations (i.e., a correlation in terms of correlation matrix R and correlation vector r) can be used to calculate the correlation between each residual spectrum and the spectra in library 322 for the second pass, and to calculate correlations in subsequent passes.

Table 1 includes the number of computations performed with and without the correlation matrix R for the subtraction algorithm illustrated in Fig. 5.

TABLE 1

| # of Compounds in Library | # of Computations without using Correlation Matrix | # of Computations using Correlation Matrix |
|---|---|---|
| 1,000 | 88,431,100 | 5,731,100 |
| 2,000 | 172,661,100 | 11,061,100 |
| 5,000 | 425,351,100 | 27,051,100 |
| 10,000 | 846,501,100 | 53,701,100 |

As demonstrated by Table 1, using the correlation matrix R significantly reduces the number of computations required to perform the subtraction algorithm. Specifically, using the correlation matrix R enables processor 304 to execute the subtraction algorithm without performing numerous intermediary correlation computations in each pass.

The following is a detailed mathematical description of implementing the above-described subtraction algorithm using the correlation matrix R, as described above. In the following discussion, X is the normalized spectra of all compounds in library 322, and Y is the normalized spectrum of the unknown mixture. Further, $M_j^k$ are the top T candidate models for a k-compound mix obtained at the end of pass k, where j=1:T. Moreover, $*: M_j^k$ is a set of T*T models from which the $M_j^k$ are selected for passes subsequent to the first pass (i.e., k>1). Finally, $e_j^k$ is the residual spectra obtained by subtracting $M_j^{k-1}$ from Y during pass k, and $H_j^k$ is the list of T top hits, obtained by comparing $e_j^k$ to the spectra in library 322.

The non-normalized spectrum of the unknown mixture acquired 402 by spectrometer 100 (shown in Fig. 1) can be expressed as:

$$S = \{s_1, s_2, \ldots s_m\}' \quad (11)$$

The energy of S can be calculated by:

$$Energy(S) = \sum_{i=1}^{m} s_i^2 \quad (12)$$

Using the calculated energy, spectrum S can be normalized to obtain the normalized spectrum Y of the unidentified mixture using:

$$Y_j = \frac{s_j}{\sqrt{Energy(S)}} \quad (13)$$

For the first pass of the subtraction algorithm (i.e., k=1), a dot product $r_y$ of Y with every compound in the library is computed using:

$$r_y = X'*Y \quad (14)$$

where X is the normalized spectra of all compounds in library 322.

Therefore, each element of r is given as:

$$r(i) = \sum_{l=1}^{m} X'(i,l) * Y(l,1) \quad (15)$$

To determine the T top hits, r is sorted in descending order. The T compounds with the highest values in r (i.e., the closest to 1) constitute the T top hits.

At the end of the first pass, the T top hits are $H_j^1$ . . Further, $H_j^1$ are the same as $M_j^1$, the top T one-compound candidate models.

For subsequent passes (i.e., k>1), the following computations are performed. For the second pass (i.e., k=2) $*M_j^k$ is initialized to the empty set. The unknown spectra Y is regressed against model $M_j^{k-1}$, and regression coefficients are computing using a least square method. The regression coefficients are represented as $b_i$, where:

$$i \in M_j^k \quad (16)$$

The correlation $r_j^k$ between the residual spectrum obtained by the subtraction of model $M_j^{k-1}$ from the unidentified spectrum and the ith compound in library 322 is computed using:

$$r_j^k = r - \sum_{i \in M_j^{k-1}} b_i R(:,i) \quad (17)$$

where $b_i$ are the regression coefficients, and $R(:,i)$ is the ith column of the pre-stored correlation matrix R.

To determine the T top hits in $_l H_j^k$, $r_j^k$ is sorted in descending order. The temporary list of model candidates (i.e., $*M_j^k$)) is generated using:

$$*M^k = *M^k \bigcup M_j^{k-1} \otimes H_j^k \quad (18)$$

where $\otimes$ is the Cartesian product operator.

To determine the top T k-compound models of the T*T models in $*M_j^k$, the mean absolute error for a given model p is calculated using:
$$(mae)_l^k = \frac{1}{m} \sum abs(Y - \sum_{i \in M_l^k} b_i X_i) \quad (19)$$

After computing the mean absolute error for each model, the T*T models are sorted by mean absolute error, and the T models with the lowest mean absolute error constitute $M_j^k$ for pass k. If the current pass is the final pass of the subtraction algorithm, the mixture is identified as the model with the lowest mean absolute error. For subsequent passes, k is incremented and the process is repeated.

Mixture Identification using a Mean Squared Error Algorithm

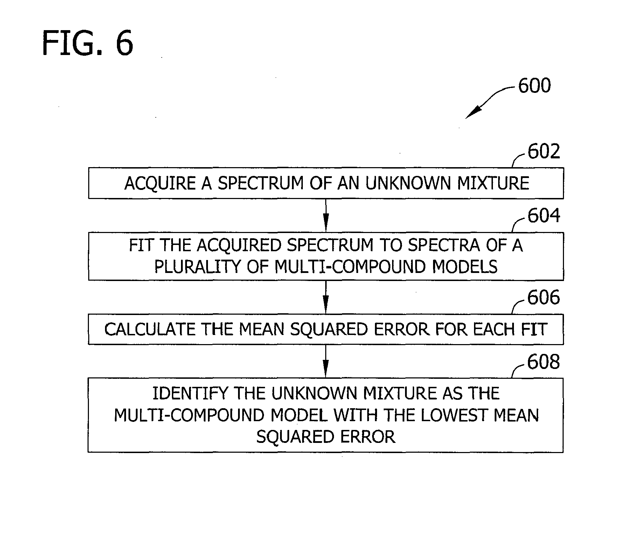

Fig. 6 is a flowchart of an exemplary method 600 for identifying an unknown mixture, such as sample 204 (shown in Fig. 2), using a mean squared error algorithm. Spectrometer 100 (shown in Fig. 1) acquires 602 a spectrum, such as a Raman spectrum, of the unknown mixture. The acquired spectrum is fit 604 to spectra of a plurality of multi-compound models, and the mean squared error is calculated 606 for each fit. The unknown mixture is identified 608 as the multi-compound model with the lowest mean squared error. Multi-compound models may be binary (i.e., two-compound) models, ternary (i.e., three-compound) models, quaternary (i.e., four-compound) models, etc. Unless otherwise noted, in the exemplary embodiment, processor 304 (shown in Fig. 3) performs the steps of method 600.

The multi-compound models are generated from combinations of the N compounds in library 322 (shown in Fig. 3). For example, a library of 700 compounds would generate roughly 250,000 binary models (i.e., roughly 250,000 possible combinations of two different compounds).

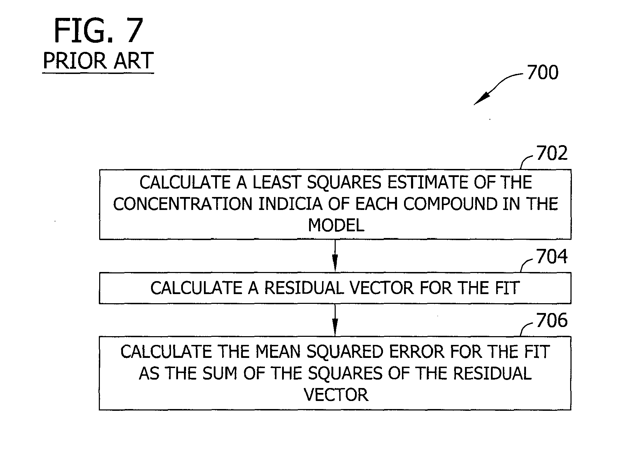

Fig. 7 is a flowchart of a known method 700 for calculating the mean squared error of each fit for a plurality of multi-compound models. For each multi-compound model, a least squares estimate of the concentration indices of each compound in the model is calculated 702. The least squares estimates are used to calculate 704 a residual vector for the fit. Finally, the mean squared error is calculated 706 as the mean of the squared terms of the residual vector. When evaluating a plurality of models, method 700 may be relatively computationally intensive.

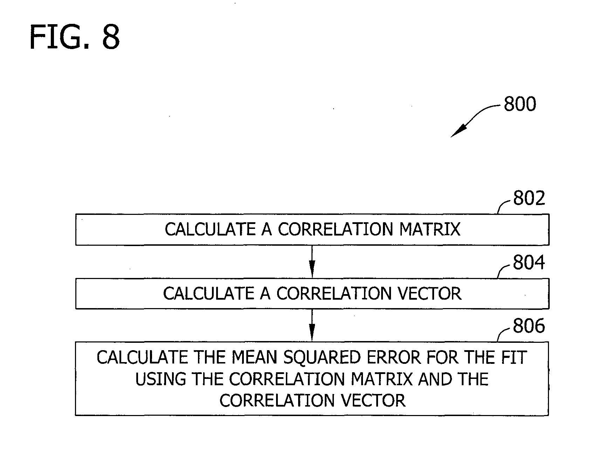

Fig. 8 is a flowchart of an exemplary method 800 for calculating the mean squared error of each fit. In contrast to method 700, method 800 utilizes a correlation matrix R and a correlation vector r to significantly reduce the number of computations needed to calculate the mean squared error of each fit. Unless otherwise noted, in the exemplary embodiment, processor 304 (shown in Fig. 3) performs the steps of method 800.

The correlation matrix R is calculated 802 from the spectra in library 322 (shown in Fig. 3). The correlation matrix R is the same correlation matrix described above in reference to the subtraction algorithm (see Equation 4).

From the spectrum y of the unknown mixture and the spectra of the compounds in library 322, the correlation vector r is calculated 804, where $r_i$ is the correlation between the spectrum of the unknown mixture and the spectrum of the ith compound in library 322. The correlation vector r is the same correlation vector described above in reference to the subtraction algorithm (see Equation 5).

Notably, the mean squared error of a particular fit can be expressed as:

$$MSE = sd \times (1 - R2) \quad (20)$$

where MSE is the mean squared error, sd is the standard deviation of the unknown mixture spectrum y, and R2 is the multivariate correlation between the unknown spectrum y and the particular compounds in the multi-compound model being fit to the unknown mixture spectrum.

Specifically, R2 can be expressed in terms of the correlation vector r and the correlation matrix R as:

$$R2 = r_{Model}^{T} * inv(R_{Model}) * r_{Model} \quad (21)$$

where $R_{Model}$ is the correlation matrix for every pair of substances in the multi-compound model under consideration and $r_{Model}^{T}$ is the transpose of the correlation vector $r_{Model}$ that is the correlation vector between the unknown spectrum and the substances in the model under consideration. $R_{Model}$ may be read from the stored correlation matrix R, or computed on the fly during execution of the algorithm. Similarly $r_{Model}$ can be read from correlation vector R or computed on the fly during execution of the algorithm.

Accordingly, the MSE of fitting a multi-compound model to the unknown mixture spectrum y can be derived in terms of the correlation vector r and the correlation matrix R. For example, for a binary model consisting of compound u and compound v, the mean squared error of the fit to the unknown mixture spectrum can be expressed as:

$$MSE = sd \times \left( 1 - \frac{r_u^2 + r_v^2 - 2r_u r_v R_{uv}}{1 - R_{uv}^2} \right) \quad (22)$$

Using Equations 20 and 21, formulas for the mean squared error for ternary (i.e., three-component) models and quaternary (i.e., four-component) models can be similarly derived.

Accordingly, with the correlation matrix R, the correlation vector r, and the energy sy of the unknown mixture spectrum y calculated, the mean squared error of the fit for each multi-compound model can be calculated 806 in relatively few computations. Specifically, by using the correlation matrix R and the correlation vector r, several of the floating point computations required in method 700 are avoided. Once the mean squared error for each multi-compound model is calculated 806, the unknown mixture is identified 608. Further, while in the exemplary embodiment, the mean squared error is calculated, alternatively, the multivariate correlation R2 by itself may be used to evaluate the multi-compound models (i.e., without calculating MSE from R2).

For both the subtraction algorithm and the mean squared error algorithm, the correlation matrix R is the same, regardless of the mixture being analyzed. Accordingly, the correlation matrix R may be calculated a single time and stored in memory 306 (shown in Fig. 3). This pre-calculated correlation matrix R may then be utilized in any number of mixture analyses.

In one embodiment, processor 304 (shown in Fig. 3) calculates the correlation matrix R during a start-up (i.e. boot sequence) of processor 304. Alternatively, the correlation matrix R may be loaded into memory 306 from another device. In yet another alternative embodiment, only a pertinent portion of correlation matrix R is calculated by processor 304 and/or loaded into memory 306 at one time. Further, the correlation matrix R may be updated as compounds are added and/or removed from library 322 (shown in Fig. 3).

The above-described embodiments utilize a correlation matrix to identify a mixture. By utilizing a correlation matrix, the number of calculations required by a mixture identification algorithm may be significantly reduced, enabling identification of mixtures in less time and with fewer computational resources. That is, floating point and/or intermediary computations required by at least some known mixture identification algorithms can be eliminated by using the correlation matrix. For example, the embodiments described herein may enable a processor to analyze an unknown mixture spectrum fifty to one-hundred times faster than at least some known algorithms. Accordingly, the embodiments described herein provide relatively efficient and fast analysis of mixtures.

A technical effect of the systems and methods described herein includes at least one of: (a) receiving a spectrum of a mixture; (b) calculating a correlation vector that includes a correlation between the mixture spectrum and each of a plurality of spectra stored in a library, each library spectrum associated with a respective compound; and (c) identifying the mixture based on the correlation vector and a correlation matrix that includes a correlation between each possible pair of spectra in the library.

A computer, such as those described herein, includes at least one processor or processing unit and a system memory. The computer typically has at least some form of computer readable media. By way of example and not limitation, computer readable media include computer storage media and communication media. Computer storage media include volatile and nonvolatile, removable and nonremovable media implemented in any method or technology for storage of information such as computer readable instructions, data structures, program modules, or other data. Communication media typically embody computer readable instructions, data structures, program modules, or other data in a modulated data signal such as a carrier wave or other transport mechanism and include any information delivery media. Those skilled in the art are familiar with the modulated data signal, which has one or more of its characteristics set or changed in such a manner as to encode information in the signal. Combinations of any of the above are also included within the scope of computer readable media.

Exemplary embodiments of methods and systems for identifying a mixture are described above in detail. The methods and systems are not limited to the specific embodiments described herein, but rather, components of systems and/or steps of the methods may be utilized independently and separately from other components and/or steps described herein. For example, the use of a correlation matrix to reduce the calculations required for a given algorithm is not limited to applications involving spectral identification. A correlation matrix could be similarly implemented in, for example, genetic search algorithms. Accordingly, the exemplary embodiment can be implemented and utilized in connection with many other applications not specifically described herein.

Although specific features of various embodiments of the invention may be shown in some drawings and not in others, this is for convenience only. In accordance with the principles of the invention, any feature of a drawing may be referenced and/or claimed in combination with any feature of any other drawing.

This written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

**Claims**

1. A spectrometer for identifying a mixture, said spectrometer comprising:

   a detector configured to generate a signal based on an interaction of light with a sample of the mixture;
   a memory device having a library and a correlation matrix stored therein, wherein the library includes a plurality of spectra, each spectrum associated with a respective compound, and wherein the correlation matrix includes a correlation between each possible pair of spectra in the library; and
   a processor coupled to said memory device and configured to:

   determine a spectrum of the mixture based on the signal generated by said detector;
   calculate a correlation vector that includes a correlation between the mixture spectrum and each spectrum in the library; and
   identify the mixture based on the correlation matrix and the correlation vector.

2. A spectrometer in accordance with Claim 1, wherein the correlation matrix is computed by a remote computing device and loaded onto said memory device, and wherein said processor is configured to identify the mixture using at least one of a covariance matrix and standard deviations of spectra in the library, wherein at least one of the covariance matrix and the standard deviations are stored in said memory device.

3. A spectrometer in accordance with Claim 1 or 2, wherein to identify the mixture, said processor is configured to:

   rank elements of the correlation vector to generate a top hit set that includes a number of compounds that are most closely correlated with the mixture;
   generate a residual spectrum for each compound in the top hit set;

calculate a correlation between each residual spectrum and each spectrum in the library using the correlation matrix and the correlation vector;

generate a residual top hit set for each residual spectrum;

produce a plurality of two-compound models from the top hit set and each residual top hit set;

rank the two-compound models according to a mean absolute error of each two-compound model; and

identify the mixture as the two-compound model with the lowest mean absolute error.

4. A spectrometer in accordance with any of Claims 1 to 3, wherein to identify the mixture, said processor is configured to:

fit the mixture spectrum to a plurality of spectra each associated with a multi-compound model;

calculate the mean squared error for each fit using the correlation matrix and the correlation vector; and

identify the mixture as the multi-compound model associated with the lowest mean squared error.

5. A spectrometer in accordance with any of Claims 1 to 4, wherein said processor is configured to fit the mixture spectrum to spectra associated with two-compound models, and wherein said processor is configured to calculated the mean squared error as $MSE = sd \times \left(1 - \dfrac{r_u^2 + r_v^2 - 2r_u r_v R_{uv}}{1 - R_{uv}^2}\right)$, where MSE is the mean squared error, sd is the standard deviation of the mixture spectrum, $r_u$ is the correlation between the mixture spectrum and the spectrum of compound u, $r_v$ is the correlation between the mixture spectrum and the spectrum of compound v, and $R_{uv}$ is the correlation between the spectrum of compound u and the spectrum of compound v from the correlation matrix.

6. A processing device configured to:

acquire a spectrum of a mixture;

calculate a correlation vector that includes a correlation between the mixture spectrum and each of a plurality of spectra stored in a library; and

identify the mixture based on the correlation vector and a correlation matrix that includes a correlation between each possible pair of spectra in the library.

7. A processing device in accordance with Claim 6, wherein said processing device is further configured to:

calculate the correlation matrix; and

update the correlation matrix when at least one new spectrum is added to the library.

8. A processing device in accordance with Claim 6 or 7, wherein to identify the mixture, said processing device is configured to:

rank elements of the correlation vector to generate a top hit set that includes a number of compounds that are most closely correlated with the mixture;

generate a residual spectrum for each compound in the top hit set;

calculate a correlation between each residual spectrum and each spectrum in the library using the correlation matrix and the correlation vector;

generate a residual top hit set for each residual spectrum;

produce a plurality of two-compound models from the top hit set and each residual top hit set;

rank the two-compound models according to a mean absolute error of each two-compound model; and

identify the mixture as the two-compound model with the lowest mean absolute error.

9. A processing device in accordance with any of Claims 6 to 8, wherein to identify the mixture, said processing device is configured to:

fit the mixture spectrum to a plurality of spectra each associated with a multi-compound model;

calculate the mean squared error for each fit using the correlation matrix and the correlation vector; and

identify the mixture as the multi-compound model associated with the lowest mean squared error.

**10.** A method for identifying a mixture, said method comprising:

acquiring, using a spectrometer, a spectrum of the mixture;
calculating, using a processing device, a correlation vector that includes a correlation between the mixture spectrum and each of a plurality of spectra stored in a library, each library spectrum associated with a respective compound; and
identifying, using the processing device, the mixture based on the correlation vector and a correlation matrix that includes a correlation between each possible pair of spectra in the library.

**11.** A method in accordance with Claim 10, further comprising:

calculating the correlation matrix; and
updating the correlation matrix when at least one new spectrum is added to the library.

**12.** A method in accordance with Claim 10 or 11, wherein identifying the mixture comprises:

ranking elements of the correlation vector to generate a top hit set that includes a number of compounds that are most closely correlated with the mixture;
generating a residual spectrum for each compound in the top hit set;
calculating a correlation between each residual spectrum and each spectrum in the library using the correlation matrix and the correlation vector;
generating a residual top hit set for each residual spectrum;
producing a plurality of two-compound models from the top hit set and each residual top hit set;
ranking the two-compound models according to a predetermined criteria; and
identifying the mixture as one of the two-compound models based on the ranking.

**13.** A method in accordance with Claim 12, wherein ranking the two-compound models comprises ranking the two-compound models according to a mean absolute error of each two-compound model, and wherein identifying the mixture comprises identifying the mixture as the two-compound model with the lowest mean absolute error.

**14.** A method in accordance with any of Claims 10 to 13, wherein identifying the mixture comprises:

fitting the mixture spectrum to a plurality of spectra each associated with a multi-compound model;
calculating the mean squared error for each fit using the correlation matrix and the correlation vector; and
identifying the mixture as the multi-compound model associated with the lowest mean squared error.

**15.** A method in accordance with Claim 14, wherein fitting the mixture spectrum comprises fitting the mixture spectrum to spectra associated with two-compound models, and wherein calculating the mean squared error comprises calculating the mean squared error using $MSE = sd \times \left(1 - \dfrac{r_u^2 + r_v^2 - 2r_u r_v R_{uv}}{1 - R_{uv}^2}\right)$, , where MSE is the mean squared error, sd is the standard deviation of the mixture spectrum, $r_u$ is the correlation between the mixture spectrum and the spectrum of compound u, $r_v$ is the correlation between the mixture spectrum and the spectrum of compound v, and $R_{uv}$ is the correlation between the spectrum of compound u and the spectrum of compound v from the correlation matrix.

# FIG. 1

# FIG. 2

200

| 202 | → ( 204 )

206

208

210

212

FIG. 3

# FIG. 4

$\diagup$ 400

402

| ACQUIRE A SPECTRUM OF AN UNKNOWN MIXTURE |
|---|

404

| COMPARE THE SPECTRUM AGAINST LIBRARY SPECTRA |
|---|

406

| GENERATE A TOP HIT SET |
|---|

408

| GENERATE A RESIDUAL SPECTRUM FOR EACH COMPOUND IN THE TOP HIT SET |
|---|

410

| COMPARE EACH RESIDUAL SPECTRUM AGAINST LIBRARY SPECTRA |
|---|

412

| GENERATE A RESIDUAL TOP HIT SET FOR EACH RESIDUAL SPECTRUM |
|---|

414

| PRODUCE TWO-COMPOUND MODELS |
|---|

416

| RANK THE TWO-COMPOUND MODELS |
|---|

418

| GENERATE ADDITIONAL RESIDUAL SPECTRA FOR EACH TWO-COMPOUND MODEL |
|---|

420

| COMPARE EACH ADDITIONAL RESIDUAL SPECTRUM AGAINST THE SPECTRA IN THE LIBRARY |
|---|

422

| GENERATE AN ADDITIONAL RESIDUAL TOP HIT SET FOR EACH ADDITIONAL RESIDUAL SPECTRUM |
|---|

424

| PRODUCE THREE-COMPOUND MODELS |
|---|

426

| RANK THE THREE-COMPOUND MODELS |
|---|

428

| IDENTIFY THE UNKNOWN MIXTURE AS THE TOP RANKED THREE-COMPOUND MODEL |
|---|

# FIG. 5

SOLUTION: | • o58-o67-o11 |

# FIG. 6

600

602

```
ACQUIRE A SPECTRUM OF AN UNKNOWN MIXTURE
```

604

```
FIT THE ACQUIRED SPECTRUM TO SPECTRA OF A
PLURALITY OF MULTI-COMPOUND MODELS
```

606

```
CALCULATE THE MEAN SQUARED ERROR FOR EACH FIT
```

608

```
IDENTIFY THE UNKNOWN MIXTURE AS THE
MULTI-COMPOUND MODEL WITH THE LOWEST MEAN
SQUARED ERROR
```

# FIG. 7
PRIOR ART

— 700

— 702

CALCULATE A LEAST SQUARES ESTIMATE OF THE
CONCENTRATION INDICIA OF EACH COMPOUND IN THE
MODEL

— 704

CALCULATE A RESIDUAL VECTOR FOR THE FIT

— 706

CALCULATE THE MEAN SQUARED ERROR FOR THE FIT
AS THE SUM OF THE SQUARES OF THE RESIDUAL
VECTOR

# FIG. 8

800

802
| CALCULATE A CORRELATION MATRIX |
| --- |

804
| CALCULATE A CORRELATION VECTOR |
| --- |

806
| CALCULATE THE MEAN SQUARED ERROR FOR THE FIT USING THE CORRELATION MATRIX AND THE CORRELATION VECTOR |
| --- |